# EUROPÄISCHE PATENTSCHRIFT

(11) **EP 0 573 850 B1**
(45) Veröffentlichungstag und Bekanntmachung des Hinweises auf die Patenterteilung: **23.12.1998**
(21) Anmeldenummer: 93108561.7
(22) Anmeldetag: 27.05.1993
(51) Int. Cl.: C07D 323/06

(54) **Verfahren zur Herstellung von reinem Trioxan**
Process for the preparation of pure trioxane
Procédé de préparation de trioxanne pure

(30) Priorität: 06.06.1992 DE 4218735
(43) Veröffentlichungstag der Anmeldung: 15.12.1993
(73) Patentinhaber: Ticona GmbH, 65451 Kelsterbach (DE)
(72) Erfinder: Mück, Karl-Friedrich, Dr., W-6200 Wiesbaden (DE); Schlaf, Helmut, Dr., W-6233 Kelkheim/Ts (DE); Rittner, Siegbert, Dr., W-6082 Mörfelden (DE)

(56) Entgegenhaltungen:
- US-A- 3 519 650
- CHEMICAL ABSTRACTS, Band 74, Nr. 14, 05 April 1971, Columbus, OH (US); Seite 16, AN 64634v

## Beschreibung

Die Erfindung bezieht sich auf ein Verfahren zur Herstellung von reinem Trioxan durch ein Kristallisationsverfahren, bei dem alkalische organische Verbindungen zugesetzt werden.

Nach dem Stand der Technik werden bei der Herstellung von Trioxan eine Vielzahl von Schritten angewandt. So wird es für gewöhnlich durch Erhitzen von 30 - 70%igen wäßrigen Formaldehydlösungen in Gegenwart von sauren Katalysatoren, z.B. 2 - 25 % Mineralsäure (DE-B 1 543 390), oder in Gegenwart von sauren Ionenaustauschern (DE-C 1 135 491) hergestellt und anschließend destillativ aus dem Reaktionsgemisch entfernt. Dies geschieht entweder in einer dem Reaktor aufgesetzten Kolonne (US-A 2 304 080) oder in einer separaten Kolonne (GB-B 1 012 372). Das trioxanreiche Destillat wird mit Methylenchlorid, Ethylenchlorid, Benzol oder Toluol extrahiert (DE-B 1 668 867), der Extrakt anschließend neutralisiert und destillativ gereinigt (DE-B 1 543 815).

Die erwähnte Extraktion ist notwendig, um die Abtrennung des Trioxans vom Wasser zu erreichen, während die genannte destillative Reinigung erforderlich ist, um bei der Trioxansynthese entstehende Nebenprodukte, wie Formaldehyd, Methanol, Ameisensäure, Methylformiat, Methylal, Dioxymethylendimethylether und andere, zu entfernen und so zu einem polymerisationsfähigen Trioxan zu kommen.

Es ist auch bekannt, die Abtrennung von Verunreinigungen im Trioxan durch Extraktivdestillation in Gegenwart von Wasser oder Ethylenglykol zu erreichen (US-A 3 281 336). Ebenfalls ist ein Verfahren bekannt, bei dem vor, während oder nach der destillativen Reinigung tertiäre Amine zugesetzt werden, die in Trioxan löslich sowie unter den gegebenen Verfahrensbedingungen stabil und nicht flüchtig sind (US-A 3 519 650).

Es ist auch möglich, zur Abtrennung der Verunreinigungen flüssiges Trioxan in einem offenen System zu kristallisieren und Luft oder ein Inertgas eventuell unter erhöhtem Druck, darüber zu bewegen (DE-A 2 885 710). Durch Überleiten von Luft können aber neue Verunreinigungen wie Peroxide im Trioxan gebildet werden.

Außerdem werden nach dieser Verfahrenweise überwiegend nur die leicht siedenden Verunreinigungen entfernt. Schwersieder, welche die Polymerisation ebenfalls stören können, verbleiben noch in signifikanten Mengen im Produkt. Nachteilig wirkt sich auch der hohe Sublimationsdruck des Trioxans bei dem geschilderten Verfahren in der Weise aus, daß Materialverluste entstehen.

In der DE-A 3 508 668 wird die Herstellung von polymerisationsfähigem Trioxan in hoher Reinheit durch mehrstufige Kristallisation beschrieben. Es ist nun beobachtet worden, daß bei einem Trioxangehalt über 95 % nach vielen Betriebsstunden oder Kristallisationszyklen eine Flusenbildung eintreten kann, die so massiv ist, daß die gesamte Kristallisationsanlage verstopft wird.

Es bestand daher die Aufgabe, ein Verfahren zur Trioxankristallisation bereitzustellen, das einerseits polymerisationsfähiges Trioxan in hoher Reinheit erzeugt und andererseits eine Flusenbildung verhindert.

Die Erfindung betrifft somit ein Verfahren zur Herstellung von hochreinem Trioxan durch Kristallisation eines Trioxangemisches, dadurch gekennzeichnet, daß dem Trioxangemisch während der Kristallisation alkalische organische Verbindungen zugesetzt werden. Aus dem erhaltenen Reintrioxan sind Polymerisate mit hohem Molekulargewicht durch kationische Polymerisation herstellbar.

Die Kristallisation des Trioxans kann nach verschiedenen Methoden, die in der Literatur bekannt sind, und in verschiedenen Arten von Kristallisatoren vorgenommen werden.
Wesentlich ist, daß die Kristallisation von mindestens einem, einem Wasch- oder Schwitzvorgang entsprechenden Verfahrensschritt begleitet ist, der die zugegebenen Komponenten simultan aus dem Kristallisat entfernt. Geeignet einem Wasch- oder Schwitzvorgang entsprechenden Verfahrensschritt begleitet ist, der die zugegebenen Komponenten simultan aus dem Kristallisat entfernt. Geeignet für das Verfahren gemäß der Erfindung sind kontinuierliche, diskontinuierliche und semikontinuierliche Kristallisationsverfahren. Besonders geeignet sind kontinuierliche Kristallisationsverfahren, wie sie in der EP-B 0 105 524 und semikontinuierliche Kristallisationsverfahren, wie sie in der DE-A 3 508 668 beschrieben sind und auf die hiermit Bezug genommen wird. Die Kristallisation kann - in Abhängigkeit von dem Reinheitsgrad des eingesetzten Trioxans und gegebenenfalls der verwendeten Apparatur - einstufig oder mehrstufig vorgenommen werden.

Es ist wesentlich, die alkalische organische Zusatzkomponente dem Prozeß in der Kristallisationsstufe oder in den Apparateabschnitt zuzugeben, in dem die Reinheit des Trioxans einem bestimmten Grad erreicht hat. Dieser liegt im allgemeinen im Bereich von 95 bis 99,9 Gew.-%, vorzugsweise 98 bis 99,9 und insbesondere 99,3 bis 99,9 Gew.-%.

Als alkalische organische Komponente eignen sich im vorliegenden Verfahren Amine. Bevorzugt werden tertiäre Amine mit gegenüber Trioxan deutlich höherem Siedepunkt eingesetzt. Hier sind vor allem Triethanolamin, Tripropylamin, Methyldiethanolamin und Dimethylethanolamin und Gemische davon, insbesondere aber Triethanolamin zu nennen.

Die zugegebene Aminmenge beträgt im allgemeinen 500 bis 5 ppm, bezogen auf Reintrioxan, bevorzugt ist der Konzentrationsbereich von 100 bis 5 ppm und insbesondere von 25 bis 5 ppm.

Die eingesetzten Amine verhindern die Flusenbildung während des Kristallisiervorgangs. Sie werden bei den Reinigungsschritten des Verfahrens (Schwitzen, Waschen) wieder soweit entfernt, daß sie analytisch nicht mehr nachweisbar sind und somit die Polymerisationsfähigkeit des Reintrioxans nicht beeinträchtigt wird.

### Beispiele

Die Beispiele 2 und 3 sowie das Vergleichsbeispiel wurden nach folgendem Verfahren durchgeführt:
In einem feuchtefreien, mit Stickstoff inertisierten Röhrenkristallisator (Winnacker-Küchler, Chemische Technologie, 4. Aufl., Band 6 (1982) S. 148), dessen Mantel an einen Thermostaten mit einem Temperatur-Zeitplangeber angeschlossen war, wurde aufgeschmolzenes, 94 %iges Trioxan (enthält außerdem Wasser, Formaldehyd und Methanol, sowie die vorstehend genannten weiteren Nebenprodukte) vorgelegt und durch Kristallisation gereinigt. Hierzu wurde das Produkt innerhalb von 30 Minuten auf 50°C abgekühlt und der Flüssigkeitsanteil abgelassen. Die im Apparat verbleibenden Kristalle, die einen Reinheitsgrad von über 95 % aufweisen, wurden aufgeschmolzen und einem weiteren gleichartigen Kristallisationsverfahren unterzogen. Im allgemeinen weist das auf diese Weise zweistufig gereinigte 94 %ige Trioxan einen Reinheitsgrad von über 99,9 % auf und ist somit für eine Polymerisation im üblichen Sinne geeignet.
1. (Vergleichsbeispiel)
   Diese Verfahrensweise wurde kontinuierlich über 24 Stunden betrieben. Nach diesem Zeitpunkt traten Polymerflusen im geschmolzenen Trioxan auf, die sehr voluminös waren und Auslaufventile und Verteilerböden der Kristallisationsapparatur verstopften. Die Gesamtmenge der Flusen lag bei 1 Gew.- %, bezogen auf Trioxan.
2. Triethanolaminzugabe im 2. Kristallisationsschritt (20 ppm, bezogen auf Trioxan)
   Gibt man bei der kontinuierlich betriebenen Verfahrensweise 20 ppm, bezogen auf Trioxan, Triethanolamin dem Kristallisationsschritt bei einem Trioxangehalt von über 95 % hinzu, so treten während einer Versuchsdauer von 120 Stunden keine Flusen auf.
3. Triethanolaminzugabe im 2. Kristallisationsschritt (100 ppm, bezogen auf Trioxan)
   Die Triethanolaminmenge wird gegenüber Beispiel 2 auf 100 ppm erhöht. Eine Flusenbildung trat während der Versuchsdauer von 120 Stunden nicht auf.

Das aus den Versuchen 1 bis 3 erhaltene hochreine Trioxan wurde gegebenenfalls von den Flusen filtriert (Vergleichsversuch 1) und jeweils mit 3,4 Gew.-% Dioxolan vermischt. In einer verschlossenen Aluminiumtube wurden die Monomerenmischungen in einem Flüssigkeitsbad auf 80°C temperiert und BF₃-etherat, verdünnt in Cyclohexan, mittels einer Injektionsspritze zu den drei Proben zugegeben. Das Monomer und der Initiator wurden durch Schütteln innig vermischt. Bestimmt wurde außer der Induktionsperiode bis zur eintretenden Polymerisation die Ausbeute an Polymer und der Schmelzindex des jeweiligen Polymerisats.

Die Ergebnisse sind in der Tabelle zusammengestellt.

Polymerisation von Trioxan in Tuben (nach Reinigung durch Kristallisation) bei 80°C in Gegenwart von BF₃-etherat (≙ 13 ppm BF₃)

| Beispiel | Aminzugabe während der Kristallisation | Induktionsperiode (sec.) | Schmelzindex MFI DIN 53735 | Ausbeute % | Amingehalt ppm |
|---|---|---|---|---|---|
| 1. Vergleich | | 53 | 2,6 | 95 | - |
| 2. | + 20 ppm | 52 | 2,8 | 94 | < 5 |
| 3. | + 100 ppm | 55 | 2,4 | 93 | < 5 |

## Patentansprüche

1. Verfahren zur Herstellung von hochreinem Trioxan durch Kristallisation eines Trioxangemisches, dadurch gekennzeichnet, daß dem Trioxangemisch eine alkalische organische Verbindung jeweils der Kristallisationsstufe oder dem Apparateabschnitt zugegeben wird, in dem die Reinheit des Trioxans bei 95 bis 99,9 Gew.-% liegt und wobei die Kristallisation von mindestens einem, einem Wasch- oder Schwitzvorgang entsprechenden Verfahrensschritt begleitet ist, der die zugegebenen Komponenten simultan aus dem Kristallisat entfernt.

2. Verfahren nach Anspruch 1, dadurch gekennzeichnet, daß die Kristallisation einstufig oder mehrstufig durchgeführt wird.

3. Verfahren nach Anspruch 1 oder 2, dadurch gekennzeichnet, daß als organische alkalische Verbindung tertiäre Amine mit gegenüber Trioxan deutlich höherem Siedepunkt eingesetzt werden.

4. Verfahren nach Anspruch 3, dadurch gekennzeichnet, daß Triethanolamin, Tripropylamin, Methyldiethanolamin und Dimethylethanolamin und Gemische davon, insbesondere aber Triethanolamin, eingesetzt werden.

5. Verfahren nach einem oder mehreren der Ansprüche 1 bis 4, dadurch gekennzeichnet, daß die alkalische organische Verbindung in der Kristallisationsstufe oder in den Apparateabschnitt zugeben wird, in dem die Reinheit des Trioxans bei 98 bis 99,9 Gew.-%, insbesondere bei 99,3 bis 99,9 Gew.-%, liegt.

6. Verfahren nach einem oder mehreren der Ansprüche 1 bis 5, dadurch gekennzeichnet, daß der Amingehalt 5 bis 500 ppm, vorzugsweise 5 bis 100 und insbesondere 5 bis 25 ppm, bezogen auf Reintrioxan beträgt.

## Claims

1. A process for the preparation of highly pure trioxane by crystallization of a trioxane mixture, which comprises adding an alkaline organic compound to the trioxane mixture in each case in the crystallization stage or in the apparatus section in which the purity of the trioxane is 95 to 99.9 % by weight and where the crystallization is accompanied by at least one process step corresponding to a washing or sweating operation which simultaneously removes the added components from the crystallizate.

2. The process as claimed in claim 1, wherein the crystallization is carried out in one or more steps.

3. The process as claimed in claim 1 or 2, wherein the organic alkaline compound employed is a tertiary amine having a distinctly higher boiling point compared to trioxane.

4. The process as claimed in claim 3, wherein triethanolamine, tripropylamine, methyldiethanolamine and dimethylethanolamine and mixtures thereof, but in particular triethanolamine, are employed.

5. The process as claimed in one or more of claims 1 to 4, wherein the alkaline organic compound is added in the crystallization step or in the apparatus section in which the purity of the trioxane is 98 to 99.9 % by weight, in particular 99.3 to 99.9 % by weight.

6. The process as claimed in one or more of claims 1 to 5, wherein the amine content is 5 to 500 ppm, preferably 5 to 100 and in particular 5 to 25 ppm, relative to pure trioxane.

## Revendications

1. Procédé de préparation de trioxanne de haute pureté par cristallisation d'un mélange de trioxanne, caractérisé en ce que l'on ajoute au mélange de trioxanne un composé organique alcalin respectivement à l'étape de cristallisation ou à la section d'appareil, dans lequel la pureté du trioxanne se situe de 95 à 99,9% en poids et dans lequel la cristallisation s'accompagne d'au moins une étape de procédé correspondant à un processus de lavage ou à un processus de ressuage, qui sépare simultanément les deux composés ajoutés du cristallisat.

2. Procédé suivant la revendication 1, caractérisé en ce que la cristallisation est réalisée en une ou en plusieurs étapes.

3. Procédé suivant la revendication 1 ou 2, caractérisé en ce que des amines tertiaires ayant un point d'ébullition nettement supérieur au trioxanne sont utilisées comme composé organique alcalin.

4. Procédé suivant la revendication 3, caractérisé en ce qu'on utilise la triéthanolamine, la tripropylamine, la méthyldiéthanolamine et la diméthyléthanolamine et des mélanges de celles-ci, en particulier cependant la triéthanolamine.

5. Procédé suivant l'une ou plusieurs des revendications 1 à 4, caractérisé en ce que le composé organique alcalin est ajouté dans l'étape de cristallisation ou dans la section d'appareil, dans lequel la pureté du trioxanne se situe de 98 à 99,9% en poids, en particulier de 99,3 à 99,9% en poids.

6. Procédé suivant l'une ou plusieurs des revendications 1 à 5, caractérisé en ce que la teneur en amine par rapport au trioxanne pur s'élève de 5 à 500 ppm, de préférence de 5 à 100 et en particulier de 5 à 25 ppm.
